# DEMANDE DE BREVET EUROPEEN

(11) **EP 2 233 115 A1**
(43) Date de publication de la demande: **29.09.2010**
(21) Numéro de dépôt: 09156021.9
(22) Date de dépôt: 24.03.2009
(51) Int. Cl.: A61F 5/56

(54) **Appareil dentaire pour le réglage du positionnement du maxillaire inférieur**

(71) Demandeur: Valceschini, Michel, 1610 Châtillens/Oron (CH)
(72) Inventeur: Valceschini, Michel, 1610 Châtillens/Oron (CH)
(74) Mandataire: Ganguillet, Cyril

(57) **Abrégé**

L'armature (3) de la partie supérieure (1) de l'appareil, noyée dans la gouttière (5) du maxillaire supérieur est accrochée à l'armature (4) de la partie inférieure (2) noyée dans la gouttière (6) du maxillaire inférieur, par introduction de la barre (8) de la languette en T (7) parallèlement aux fentes (12), dans l'une de celles-ci, choisie pour l'accrochage. La fixation est ensuite réalisée par rotation relative d'un quart de tour des deux parties (1 et 2), et l'appareil est introduit dans la cavité buccale.

## Description

### Domaine technique

La présente invention a pour objet un appareil dentaire pour le réglage du positionnement du maxillaire inférieur.

### Etat de la technique

Les appareils dentaires pour le réglage du positionnement du maxillaire inférieur sont utilisés d'une part en orthodontie et d'autre part pour prévenir le ronflement et les apnées obstructives du sommeil.

On sait notamment que les manifestations pathologiques du sommeil telles que ronflement ou apnée du sommeil peuvent être prévenues en maintenant la mâchoire inférieure dans une position avancée par rapport à la mâchoire supérieure. De nombreux appareils capables de maintenir les mâchoires dans cet état ont déjà été proposés, mais ils présentent pour la plupart des défauts qui entravent leur application généralisée.

Ainsi, on connaît par la publication DE 41 260 A1 un appareil oral destiné à prévenir le ronflement et comprenant deux parties avec chacune une gouttière dentaire adaptée à l'un des maxillaires, supérieur et inférieur respectivement, gouttière dans laquelle un organe de fixation est partiellement noyé. Dans cet appareil connu, l'organe de fixation partiellement noyé dans la gouttière dentaire du maxillaire inférieur est une pièce annulaire avec une ouverture allongée parallèle à la portion de l'arc dentaire dans laquelle elle est positionnée. L'autre organe de fixation comporte un mécanisme complexe, ramassé sur lui-même et muni d'un crochet en forme de T saillant de la gouttière correspondante et relié par une transmission à glissière à une pièce de commande permettant de le déplacer sur un guidage au moyen d'un outil simple. Cet appareil est relativement peu encombrant et a l'avantage de permettre des réglages, par exemple une augmentation progressive de l'avancement du maxillaire inférieur soit lors de l'ajustage d'un appareil au moment de son acquisition soit à l'usage en fonction des résultats obtenus. Cependant, ses défauts sont d'une part la difficulté d'entretien et le manque d'hygiène du fait de l'encrassement des parties mobiles telles que la glissière et la vis de commande, et d'autre part le fait que les organes de fixation sont des parties métalliques de faible extension noyées dans les gouttières dentaires, en général en matière synthétique de sorte que la force d'avancement qui doit être transmise de l'appareil aux arcs dentaires traverse des zones où les caractéristiques mécaniques des matériaux changent brusquement ce qui offre le risque de fatigue, de fissuration ou de rupture à l'usage prolongé.

### Divulgation de l'invention

Le but de la présente invention est donc de créer un appareil qui intègre les avantages de celui auquel on fait référence ci-dessus et élimine ses défauts, notamment en considérant les résultats de certains essais qui montrent qu'une possibilité de réglage par incréments est aussi efficace que le réglage continu des appareils connus.

Dans ce but, l'appareil selon l'invention du genre défini ci-dessus est caractérisé, selon la revendication 1 annexée, en ce que les organes de fixation sont constitués l'un et l'autre d'une seule pièce métallique mince et rigide mise en forme par découpage pliage et/ou emboutissage, de forme arquée vue en plan et présentant un élément fonctionnel d'accrochage dégagé de la gouttière.

### Brève description des dessins

On décrit ci-après, à simple titre d'exemple non limitatif, une forme d'exécution de l'appareil selon l'invention, ainsi que quelques variantes, en se référant au dessin annexé dans lequel :
Les figs 1 et 2 représentent des vues en perspective des deux parties respectivement supérieure et inférieure de la première forme d'exécution,
Les figs 3 et 4 représentent des vues en plan de dessus, à échelle réduite, montrant les organes de fixation d'une variante d'exécution,
Les figs 5 et 6 représentent des vues en élévation dans le sens de leur axe central montrant les organes de fixation des figs 3 et 4,
La fig. 7 représente une vue en élévation montrant une variante d'exécution de l'organe de fixation inférieur, dans une direction perpendiculaire à son axe de symétrie, et
La fig. 8 représente une vue d'ensemble schématique de l'appareil des figs 1 et 2 lors de la manoeuvre d'engagement.

### Description détaillée de modes d'exécution de l'invention

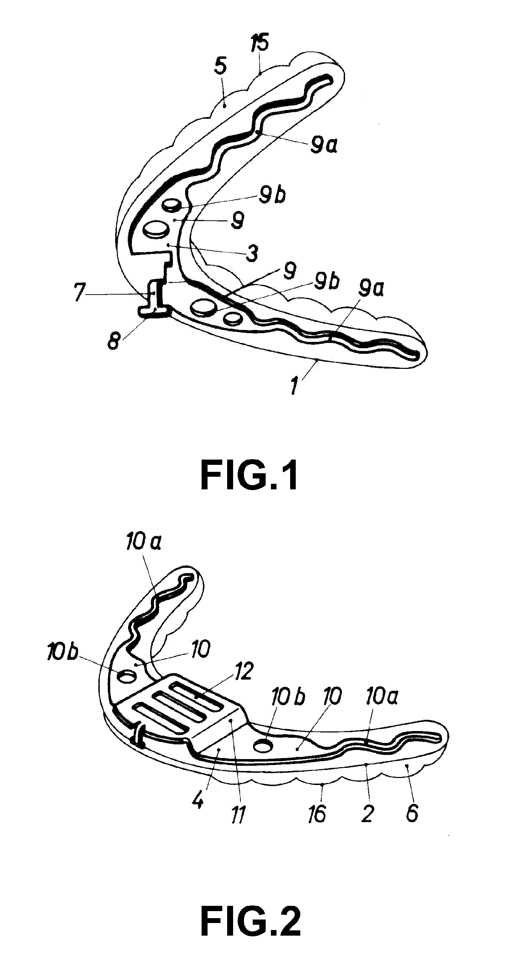

Chacune des deux parties 1 et 2 de l'appareil des figs 1 et 2 comprend un constituant métallique mince 3, 4 noyé dans une gouttière 5, 6 en une matière synthétique. Ces gouttières reproduisent en creux la forme des dentures des maxillaires supérieur et inférieur respectivement. La partie 1 est vue de bas en haut à la fig. 1, tandis que la partie 2 est vue de haut en bas à la fig. 2.

Le constituant 3 de la fig. 1 est découpé d'une pièce dans une tôle métallique d'une épaisseur d'environ un millimètre par exemple. Le métal utilisé sera un alliage compatible avec les exigences médicales, il sera notamment inoxydable, résistant aux agents chimiques, indéformable, inrayable. En outre, ses propriétés mécaniques doivent permettre les opérations de découpage, emboutissage, pliage et polissage aptes à réaliser les formes indiquées ci-après.

Le constituant 3 de la fig. 1 joue le rôle d'armature pour la partie 1 de l'appareil décrit. C'est une pièce de forme générale en V avec sommet arrondi. Une languette 7 en forme de T est découpée et pliée perpendiculairement au plan du V dans le bord extérieur arrondi de la pièce 3, la barre 8 du T étant orientée selon l'axe de symétrie central de cette pièce. L'armature 3 est découpée avec les branches 9 du V en forme d'ondulations 9a de largeur relativement étroite et, dans la zone du sommet du V, quatre ouvertures 9b disposées symétriquement. Cette disposition est destinée d'une part à limiter au maximum le poids de la pièce sans affaiblir sa résistance à la déformation et d'autre part à améliorer la cohésion entre l'armature et la gouttière 5.

Cette dernière présente des empreintes 15 des dents du maxillaire supérieur de la personne à laquelle l'appareil est destiné. En outre, elle enrobe entièrement l'armature 3 à l'exception de la languette 7. Le matériau synthétique utilisé satisfera naturellement aux exigences médicales usuelles. Techniquement, il est possible de choisir soit un matériau relativement dur, comme une résine acrylique, soit un matériau ayant une certaine souplesse d'adaptation, comme un élastomère.

Le constituant 4 de la fig. 2 joue le rôle d'armature métallique pour la partie inférieure 2 de l'appareil décrit. Sa constitution est analogue à celle de l'armature 3 avec une forme en V, des branches 10 à ondulations 10a et ouvertures 10b voisines de la partie centrale 11. Cette dernière forme une plaquette rectangulaire 11 décalée par emboutissage selon un plan parallèle à celui des branches 10 de l'armature 4. Des fentes parallèles 12 sont découpées dans le sens de la longueur de la plaquette 11, ces fentes ayant une largeur supérieure à la largeur du montant de la languette en T 7, mais inférieure à la longueur de la barre 8. Le nombre des fentes 12 n'est pas critique. Dans la forme d'exécution décrite ici, trois fentes sont prévues, mais il est évident que selon le cas l'armature 4 peut être réalisée avec un autre nombre de fentes supérieur ou inférieur à trois.

Comme pour la partie 1 de l'appareil, l'armature 4 est noyée dans une gouttière 6 de l'arc dentaire du maxillaire inférieur de la personne à laquelle l'appareil est destiné. Celle-ci présente des empreintes 16 de la denture correspondante. Cette gouttière enrobe entièrement l'armature 4 à l'exception de la plaquette 11, en laissant en outre sous les fentes 12 un espace suffisant pour que la barre 8 puisse s'y loger.

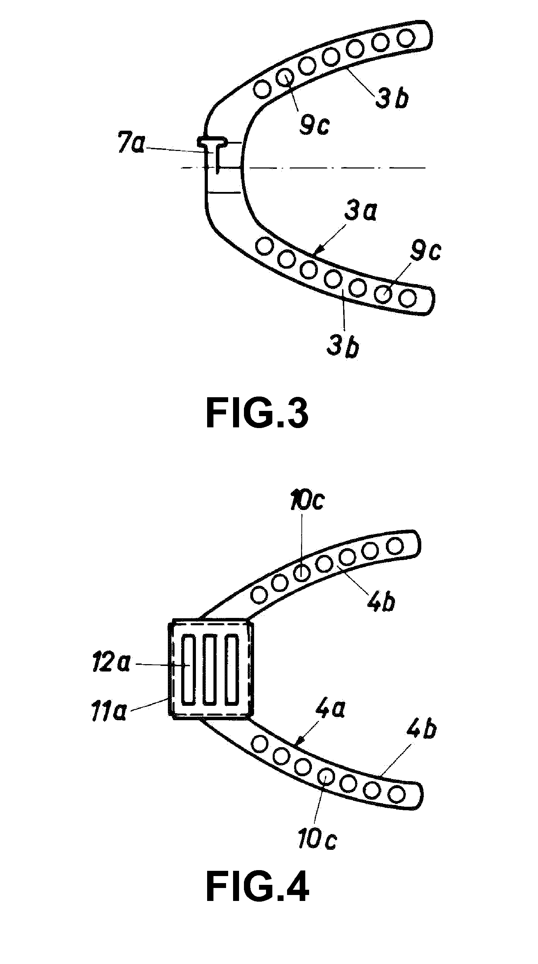

Les figs 3 à 7 montrent encore en plan de dessus et en élévation quelques variantes de forme possibles pour les armatures décrites en relation avec les figs 1 et 2.

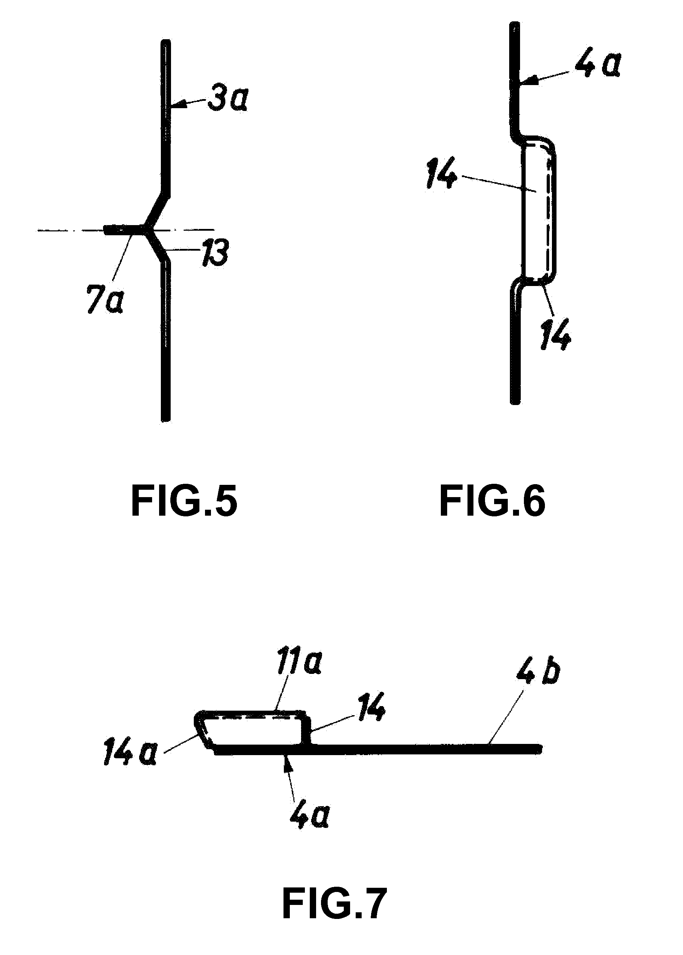

Aux figs 3 et 4, les branches latérales 3b et 4b des armatures 3a et 4a sont de largeur constante sur toute leur longueur mais présentent des ouvertures 9c et 10 c au lieu des ondulations 9a et 10a. En outre, la languette 7a découpée et pliée selon les figs 3 et 5 s'étend à partir du fond d'une dépression en V 13 emboutie dans la zone centrale de l'armature 3a.

Selon les figs 4 et 6, la plaquette 11a de l'armature 4a est de forme carrée et se raccorde avec les branches 4b de cette armature par trois parois 14 verticales et une quatrième 14a légèrement oblique qui s'étend à l'avant de l'armature comme le montre la fig. 7. Elle présente trois fentes 12a analogues aux fentes 12.

Qu'il s'agisse de la forme d'exécution représentée aux figs 1 et 2 ou des variantes représentées aux figs 3 à 7, les branches 9, 9a des constituants métalliques 3 et 4 sont susceptibles, de par leur configuration, d'être déformées facilement par le technicien de façon à adapter leur contour extérieur à la configuration de l'arcade dentaire du patient. Il en résulte que les constituants métalliques 3 et 4 peuvent avantageusement être produits en grande série dans un format standard unique.

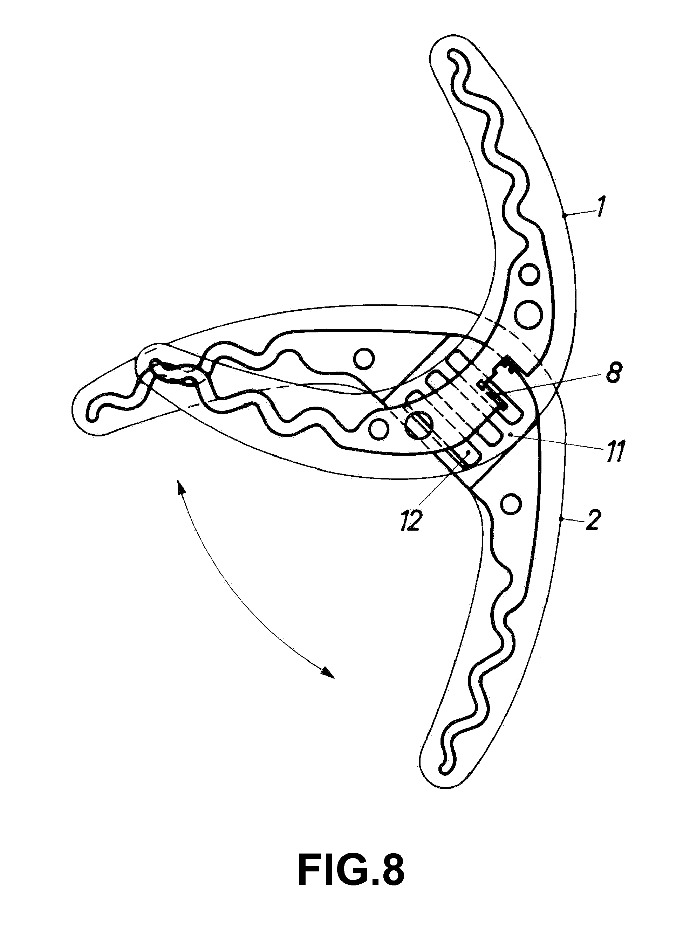

L'utilisation de l'appareil décrit aux figs 1 et 2 est représentée aux figs 8 et 9. Avant d'introduire l'appareil dans la cavité buccale, on présente ses deux parties 1 et 2 dans des positions à angle droit l'une par rapport à l'autre, comme le montre la fig. 8, de sorte que la barre 8 de la languette en T 7 peut être introduite dans une des fentes 12 de la plaquette 11. Cette fente sera choisie au préalable en fonction des effets désirés. Ensuite, il suffit de faire tourner les parties de façon à les amener en parallèle, d'accrocher la barre 8 de la languette en T dans la fente 12 choisie, et de mettre l'appareil en place dans la cavité buccale, en maintenant les gouttières l'une sur l'autre avec le pouce et l'index.

Les constituants métalliques 3 et 4 seront de préférence positionnés dans leur gouttière correspondante de façon que l'effet souhaité d'avancement du maxillaire inférieur soit obtenu, une fois l'appareil placé dans la cavité buccale du patient, lorsque la languette en T 7 est positionnée dans la première fente de la plaquette 11 située à l'avant de la partie inférieure 2 de l'appareil. Au besoin, pour augmenter encore l'avancement de la mâchoire inférieure, le patient pourra placer la languette en T 7 dans la deuxième, voire dans la troisième fente. Il convient de relever que le réglage de l'avancement par le patient lui-même, sans avoir recours au praticien ni à aucun outil, représente une innovation importante.

Dans la plupart des cas, l'appareil sera réalisé comme décrit ci-dessus, avec le constituant métallique 3 muni de la languette en T 7 disposée dans la gouttière supérieure et le constituant métallique 4 comportant la plaquette 11 munie des fentes 12 dans la gouttière inférieure.

Pour les patients présentant des cas de prognatie physiologique importante (mâchoire inférieure déjà très avancée par rapport à la mâchoire supérieure), afin d'éviter de devoir placer la languette en T 7 très en avant, en dehors de l'arcade des dents supérieures (ce qui provoquerait une protubérance poussant la lèvre supérieure), on peut inverser la position des gouttières de façon à positionner la plaquette 11 munie des fentes 12 sur la mâchoire supérieure. Dans ce cas, lors de la fabrication des gouttières 1 et 2, les constituants métalliques 3 et 4 seront positionnés à l'intérieur de leur gouttière correspondante de façon que l'effet souhaité d'avancement du maxillaire inférieur soit obtenu avec la languette T 7 positionnée dans la dernière fente de la plaquette 11, c'est-à-dire dans celle située le plus à l'intérieur de la gouttière.

Comme on l'a déjà mentionné plus haut, l'appareil selon l'invention permet avantageusement le réglage de l'avancement du maxillaire sans avoir recours au praticien, ni à aucun outil. De plus, la forme arquée des constituants métalliques 3 et 4 et la présence des deux ailes latérales 9 et 10 arquées épousant la forme de la gouttière permettent une bonne répartition de l'effort sur tout ou partie des dents et non sur les seules dents antérieures.

D'autre part, du fait de la longueur de barre 8 de la languette en T 7 sensiblement plus importante que l'épaisseur de la plaquette 11 et de la possibilité à ladite languette de se déplacer latéralement dans l'une des fentes 12, les deux gouttières ne sont pas totalement immobilisées l'une par rapport à l'autre mais peuvent jouir d'une certaine mobilité dans les trois dimensions, ce qui confère un confort très appréciable pour le patient.

## Revendications

1. Appareil dentaire pour le réglage du positionnement du maxillaire inférieur, comprenant deux parties (1, 2) avec chacune une gouttière (5, 6) adaptée à l'un des maxillaires, supérieur et inférieur respectivement, dans chacune desquelles un organe de fixation (3, 4) est partiellement noyé, **caractérisé en ce que** lesdits organes de fixation (3, 4) sont constitués l'un et l'autre d'une seule pièce métallique mince et rigide, mise en forme par découpage, pliage et/ou emboutissage, de forme arquée vue en plan et présentant un élément fonctionnel d'accrochage (7, 11), dégagé du moulage.

2. Appareil selon la revendication 1, **caractérisé en ce que** lesdits éléments fonctionnels d'accrochage (7, 11) sont de formes complémentaires, l'un d'eux (7) étant unique et saillant, l'autre (11) agencé avec plusieurs ouvertures (12), permettant un choix de fixations desdites parties dans différentes positions relatives.

3. Appareil selon la revendication 2, **caractérisé en ce que** ledit élément fonctionnel unique (7) est une languette pliée à angle droit par rapport au plan général de l'organe et ayant un contour découpé en T (7).

4. Appareil selon la revendication 3, **caractérisé en ce que** l'élément fonctionnel (11) ayant plusieurs ouvertures d'accrochage est une plaquette orientée selon le plan général de l'organe de fixation et présentant plusieurs fentes (12) découpées parallèles, de largeur telle que la languette en T (7) de l'élément fonctionnel unique puisse être introduite à choix dans l'une quelconque desdites fentes (12).

5. Appareil selon la revendication 4, **caractérisé en ce que** la disposition relative desdits éléments fonctionnels (7, 12) par rapport auxdites gouttières (5, 6) est telle que, la languette en T (7) étant introduite dans une desdites fentes (12) en ayant sa barre (8) alignée sur la fente (12), une rotation relative d'un quart de tour des parties (1, 2) de l'appareil accroche à la plaquette (11) les extrémités de la dite barre (8) du T.

6. Appareil selon la revendication 1, **caractérisé en ce que** chacun desdits organes de fixation (3, 4) présente une portion centrale (7, 11) formant ledit élément fonctionnel et deux ailes latérales (9, 10) arquées épousant la forme de la gouttière (5, 6).
